# EUROPEAN PATENT APPLICATION

(11) **EP 4 400 508 A1**
(43) Date of publication of application: **17.07.2024**
(21) Application number: 22866110.4
(22) Date of filing: 28.04.2022
(51) Int. Cl.: C07K 7/06, A61K 38/08, A61P 17/18, A61P 39/06

(54) **ACTIVE PEPTIDE AND USE THEREOF**

(30) Priority: 09.09.2021 CN 202111064688
(71) Applicant: Harvest Biotech Co., Ltd, Jiaxing, Zhejiang 314006 (CN); Yunnan Botanee Bio-Technology Group Co., Ltd., Kuming, Yunnan 650106 (CN)
(72) Inventor: LI, Junxiang, Jiaxing, Zhejiang 314006 (CN); FU, Xiaolei, Jiaxing, Zhejiang 314006 (CN); XU, Ming, Jiaxing, Zhejiang 314006 (CN); XIA, Chunxin, Jiaxing, Zhejiang 314006 (CN); KANG, Sining, Jiaxing, Zhejiang 314006 (CN); LU, Yi, Jiaxing, Zhejiang 314006 (CN)
(74) Representative: Dilg, Haeusler, Schindelmann Patentanwaltsgesellschaft mbH
(86) International application number: PCT/CN2022/090011
(87) International publication number: WO 2023/035632

(57) **Abstract**

The present disclosure relates to the field of skin repair and improvement, and in particular to an active peptide and a use thereof. The active peptide obtained in the present disclosure has the advantages of delaying senescence, anti-oxidation, anti-inflammatory properties and the like, and can effectively alleviate skin damage caused by ultraviolet irradiation.

## Description

### Cross-reference to Related Applications

The present disclosure claims priority to the Chinese patent application with the filing No. "CN202111064688.6" filed with the Chinese Patent Office on September 9, 2021, and entitled "Active Peptide and Use Thereof", the contents of which are incorporated herein by reference in entirety.

### Technical Field

The present disclosure relates to the field of skin repair and improvement, and specifically to an active peptide and related use thereof.

### Background Art

Mesenchymal stem cells (MSCs) are a class of multipotent stem cells from mesoderm in early development, and are one of the most studied and important adult stem cells at present. MSCs are widely present in a variety of tissues of the whole body, can be cultured and amplified *in vitro,* can be differentiated into a variety of cells including nerve cells, osteoblasts, chondrocytes, muscle cells, and adipocytes under specific conditions, and still have a multi-directional differentiation potential after continuous subculture and cryopreservation. Compared with MSCs from other sources, MSCs from umbilical cord have characteristics such as convenient collection, no ethical arguments, low immunogenicity, fast self-renewal, stable multiplication rate and strong proliferation capacity. Therefore, umbilical cord MSCs are suitable for clinical research and application, and are a preferred choice for cell therapy and regenerative medicine.

Statistical data from website www.ClinicalTrials.gov indicated that, as of October 2018, 705 clinical trials based on MSCs had been registered all over the world, where more than 80% of the clinical trials are in clinical phase I and phase II, and about 15% are in a stage of clinical phase III. Current experimental results indicated that MSCs are safe and effective for treatment of a variety of diseases, where types of these diseases include graft-versus-host diseases, spinal cord injuries, autoimmune diseases, cardiovascular diseases, bone and cartilage injuries and repair, Crohn's disease, diabetes and complications thereof, etc. Currently, 11 mesenchymal stem cell medicines have been approved worldwide for clinical treatment of the above diseases. However, the mechanism by which MSCs play a role in the treatment of diseases has not yet been clear, and in-depth research and exploration are still needed.

Lots of evidence indicate that mesenchymal stem cells (MSCs) may exert therapeutic effects through paracrine or autocrine, and efficacies thereof are related to angiogenesis, immunomodulation and apoptosis. MSC secretome, i.e. a sum of bioactive molecules secreted by MSCs, includes various active ingredients such as cytokines, chemokines and growth factors, and has a regulatory effect on many physiological processes such as repair, regeneration and functional rehabilitation of injured tissues. In the field of trauma care, formulations developed based on the MSC secretome are very likely to become a new therapy in place of the MSC treatment, and have a broad development prospect (WANG Qiang et al., Mesenchymal Stem Cells Secretome: A Novel Alternate Therapy for Wound Healing, CHINA BIOTECHNOLOGY, 2017, Vol. 37 Issue (4): 104-109). However, researches based on mesenchymal stem cell (MSC) secretory peptides currently are still in a primary stage, and current research and development results can hardly meet diversified requirements of consumers. Besides, prior to formal use of the MSC secretome in the clinic at present, more *in vivo* experiments and *in vitro* experiments are still required, so as to obtain a bioactive peptide with better commercialization prospects.

However, the active peptides found in nature and bodies have certain defects, for example, a relatively long peptide segment causes it unstable *in vivo,* a purification process and a synthesis process are complex, a production cost for scale production is high, and transdermal property is poor. Therefore, in order to further improve druggability and elevate commercial value, it is necessary to perform structural modification on related active peptides.

Based on the above thought, the inventors of the present disclosure, on the basis of obtaining LQ-17 active peptide through research and development, further obtained EQ-9 active peptide through structural modification. The EQ-9 bioactive peptide has activity equivalent to that of LQ-17, but since it is of a truncated structure, the production cost may be further reduced, and an unexpected technical effect is achieved.

### Summary

An objective of the present disclosure is to provide a bioactive polypeptide and a preparation method and a use thereof.

The objective of the present disclosure may be achieved by the following technical solutions.

The present disclosure provides a bioactive polypeptide, an amino acid sequence of which conforms to the following general formula:

R1-AA₁-AA₂-AA₃-AA₄-AA₅-AA₆-AA₇-AA₈-AA₉

wherein
AA₁ is selected from E and D or is a bond,
AA₂ is selected from S and T or is a bond,
AA₃ is selected from E, D, and K,
AA₄ is selected from T, A, S, and I,
AA₅ is selected from R, K, and E,
AA₆ is selected from I, L, and A,
AA₇ is selected from L, I, and V,
AA₈ is selected from L, I, and V,
AA₉ is selected from Q, E, and D or is a bond, and
R1 is selected from the group consisting of H, acetyl, tert-butanoyl, hexanoyl, 2-methylhexanone, cyclohexanecarboxyl, octanoyl, decanoyl, lauroyl, myristoyl, palmitoyl, stearoyl, oleoyl and linoleoyl.

In some embodiments, in the bioactive polypeptide conforming to the above general formula, AA₅ is R, AA₆ is I, AA₇ is L, and AA₈ is L.

In some embodiments, the amino acid sequence of the bioactive polypeptide is selected from the group consisting of ESETRILLQ, ESETKILLQ, DSDAKILIQ, DSDAKLLIQ, ETDTRILVE, ESEARIILE, DSESKLVID, ESETEAVLQ, ESKIRILLQ, ETRILL, and SETRIL.

The present disclosure provides a preparation method for the bioactive polypeptide, wherein the bioactive polypeptide may be artificially synthesized by a genetic engineering method, may be directly obtained from cells by a method of separation and purification, and more directly is prepared by chemical synthesis.

The present disclosure provides a use of the bioactive polypeptide in the preparation of food, health care products, pharmaceutical products or cosmetics having an anti-inflammatory function.

The present disclosure provides a use of the bioactive polypeptide in the preparation of food, health care products or medicines having an anti-aging function.

The present disclosure provides a use of the bioactive polypeptide in the preparation of food, health care products or medicines having an anti-oxidation function.

The present disclosure provides a product, including the bioactive polypeptide or a derivative thereof. The derivative of the bioactive polypeptide refers to a polypeptide derivative obtained through modification on an amino acid side chain group, amino terminal or carboxyl terminal of the bioactive polypeptide such as hydroxylation, carboxylation, carbonylation, methylation, acetylation, phosphorylation, esterification or glycosylation, meanwhile without affecting biological activity thereof.

The present disclosure provides a pharmaceutical composition including a cosmetically or pharmaceutically effective amount of at least one bioactive peptide according to the foregoing and at least one excipient or a cosmetically or pharmaceutically acceptable adjuvant.

In some embodiments, a dosage form of the pharmaceutical composition includes: cream, lotion, aqueous solution, gel, oil, powder, muds, wax-based forms, patches, films or freeze-dried forms.

In some embodiments, the excipient includes: hydroxyethyl cellulose, hydroxypropyl cellulose, hydroxypropyl methylcellulose, polyethylene glycol, polyacrylamide, polyacrylic acid, polyvinyl alcohol, dextran, ammonia, lactic acid, citric acid, pyrrolidone carboxylic acid, glycerol, urea, sorbitol, amino acids, lanolin, and petrolatum.

In some embodiments, the adjuvant includes: a solubilizing agent, a thickening agent, a gelling agent, a softening agent, an antioxidant, a suspending agent, a stabilizer, a foaming agent, a flavoring agent, a surfactant, water, an ionic or non-ionic emulsifier, a filler, a chelating agent, a chelating agent, a preservative, vitamin, a blocking agent, a wetting agent, essential oil, a dye, and pigment.

In addition, the bioactive peptide of the present disclosure further may be formulated with an excipient and an adjuvant commonly used in oral compositions or food supplements, such as, but not limited to, fat ingredients, aqueous ingredients, humectants, preservatives, texturizing agents, flavoring agents, fragrances, antioxidants and coloring agents commonly used in the food industry.

The bioactive peptide of the present disclosure may be used in combination with other ingredients, including, but not limited to, antioxidants, reactive carbonyl species scavengers, antiglycation agents, antihistaminic agents, antiviral agents, antiparasitic agents, emulsifiers, emollients, anti-wrinkle agents and/or anti-aging agents, etc.

The inventors have conducted extensive researches on the function of the bioactive peptide of a structure R1-AA₁-AA₂-AA₃-AA₄-AA₅-AA₆-AA₇-AA₈-AA₉, carried out a lot of experiments, determined structural domains that play an important role in the biological activity thereof, and finally obtained the bioactive peptide having good activity, a low production cost and good stability, and having multiple significant anti-aging, antioxidant and anti-inflammatory effects.

### Brief Description of Drawings

FIG. 1: chicken embryo safety experiment;
FIG. 2: cell viability assay experiment;
FIG. 3: experiment of detecting ability to protect cells from UVA damage (cell viability index);
FIG. 4: experiment of detecting ability to protect cells from UVA damage (index of collagen type I content);
FIG. 5: experiment of detecting ability to protect cells from UVA damage (index of intracellular ATP content);
FIG. 6: experiment of detecting ability to repair UVA damage (cell viability index);
FIG. 7: experiment of detecting ability to repair UVA damage (index of MMP-9 protein content);
FIG. 8: experiment of repairing UVA damage by remodeled bioactive peptide; and
FIG. 9: experiment of repairing UVA damage by EQ-9 polypeptide modified by different strategies.

### Embodiments

### Definitions

Notwithstanding numerical ranges and parameter approximations shown in broad ranges of the preset disclosure, numerical values shown in examples are described as precisely as possible. However, any numerical values inherently contain certain errors caused by standard deviation existing in their respective measurement. In addition, all ranges disclosed herein are to be understood to encompass any and all subranges covered therein. For example, a recited range of "1 to 10" should be considered to encompass any and all subranges between the minimum value 1 and the maximum value 10 (including endpoints); that is to say, all subranges starting with the minimum value 1 or greater, for example, 1 to 6.1, and ending with the maximum value 10 or less, for example, 5.5 to 10. In addition, any reference documents referred to as being "incorporated herein" should be construed as being incorporated in its entirety.

Amino acids involved in the present disclosure are represented herein by standard three letters or single-letter abbreviations.

### Related polypeptides of the present disclosure are obtained through the following procedure.

With an MSC secretome as an experimental material, polypeptides with a molecular weight of less than 30 kDa are screened by LC-MS liquid chromatography mass spectrometry. Nearly a thousand bioactive peptides are screened by a ribosome profiling technique. Further, LQ-17 is obtained by screening according to a protein-peptide interaction prediction (HSM PPI prediction) method based on machine learning and biophysics. Truncation is performed on the LQ-17 to obtain truncated bodies VQ-13, EQ-9, and LT-12, of which sequences are as follows:

**Table 1: LQ-17 Truncated Bodies**

| No. | name | sequence |
|---|---|---|
| 1 | LQ-17 | LSDQVPDTESETRILLQ |
| 2 | VQ-13 | VPDTESETRILLQ |
| 3 | EQ-9 | ESETRILLQ |
| 4 | LT-12 | LSDQVPDTESET |

### Example 1: Safety Study-Chicken Embryo Test

Experiment was carried out with reference to relevant standards and steps of Cosmetics Ocular Irritant and Corrosive HET-CAM Test (SN/T 2329-2009), and an experimental result was obtained by an irritation score (IS) method, wherein the result was: IS=0.35, being non-irritant. The evaluation criteria were as follows:

**Table 2:**

| Irritation Score | Irritation Classification |
|---|---|
| IS<1 | No Irritation |
| 1≤IS<5 | Light Irritati on |
| 5≤IS<9 | Medium Irritation |
| IS≥10 | Strong Irritation/Causticity |

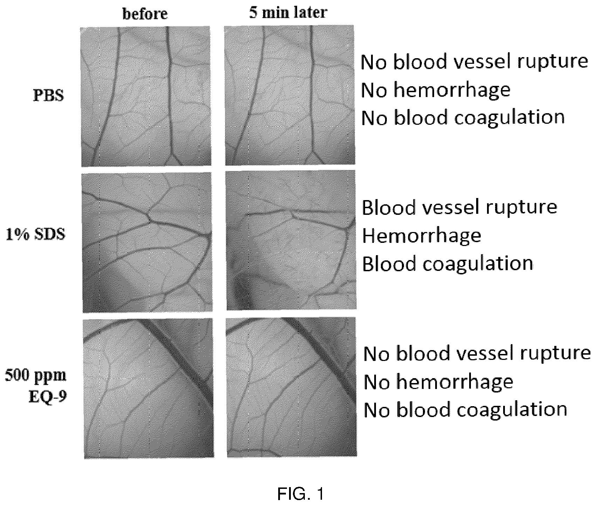

The experimental result indicates that there is no safety issue with EQ-9 at dose up to 500 ppm, with reference to FIG. 1.

### Example 2: Cell Viability Detection Experiment

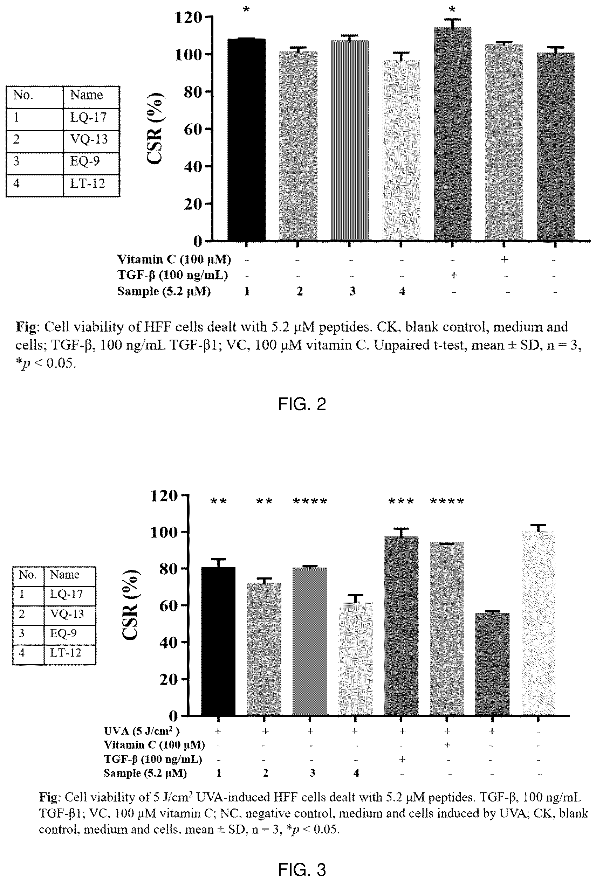

HaCaT cells were subjected to resuscitation and passage. After a state of the cells was good upon microscopic examination, the cells were collected. Cell concentration was adjusted to 0.5-1.0*10⁵/mL, and the cells were paved in a 96-well plate according to 100 µL/well. After the cells were cultured in an incubator (37°C, 5% CO₂) to a cell fusion degree of about 80%, testing substances of a certain concentration were added, followed by incubation for 24 h. Supernatant was removed, and cell viability was determined using a CCK8 detection kit. Results are shown in FIG. 2. The ability of EQ-9 to promote cell survival is obviously stronger than that of LQ-17.

### Example 3: Experiment of Detecting Ability to Protect Cells from UVA Damage

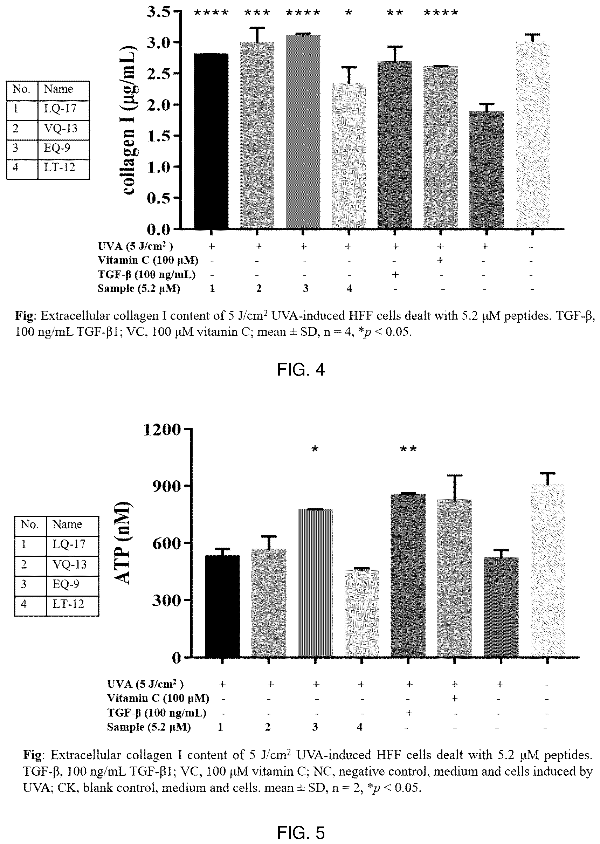

Human foreskin fibroblast HFF cells and immortalized human immortalized keratinocytes HaCaT cells were subjected to resuscitation and passage. After a state of the cells was good upon microscopic examination, the cells were collected and added to a 96-well plate according to a cell density of 2.0-2.5*10⁴/well. The cells were cultured in an incubator for 24 h (37°C, 5% CO₂). Culture medium was discarded. Culture medium containing testing substances of a certain concentration was added. Induction was performed with a certain amount of UVA, wherein a total irradiation amount was controlled to be the same and within 5-15 J/cm². Supernatant was discarded. A culture medium containing certain testing substances was added. After culturing for 24 h, cell viability, collagen content and ATP content were measured using a corresponding kit (see FIG. 3-FIG. 5). Results indicate that:
1. LQ-17 and EQ-9 may significantly improve cell survival under ultraviolet-stimulated conditions.
2. LQ-17 and EQ-9 significantly increase a content of collagen type I outside testing cells under ultraviolet-stimulated conditions.
3. Under ultraviolet-stimulated conditions, only EQ-9 can significantly increase intracellular ATP content.

### Example 4: Experiment of Testing Ability to Repair UVA Damage

Human foreskin fibroblast HFF cells were subjected to resuscitation and passage, and after a state of the cells was good upon microscopic examination, the cells were collected and added to a 96-well plate according to a cell density of 2.0-2.5*10⁴/well. The cells were cultured in an incubator for 24h (37°C, 5% CO₂). Culture medium was discarded. Fresh culture medium was added. Induction was performed with a certain amount of UVA, wherein a total irradiation amount was controlled to be 5 J/cm². Supernatant was discarded. A culture medium containing certain testing substances was added. After culturing for 24 h, cell viability was measured using a corresponding kit.

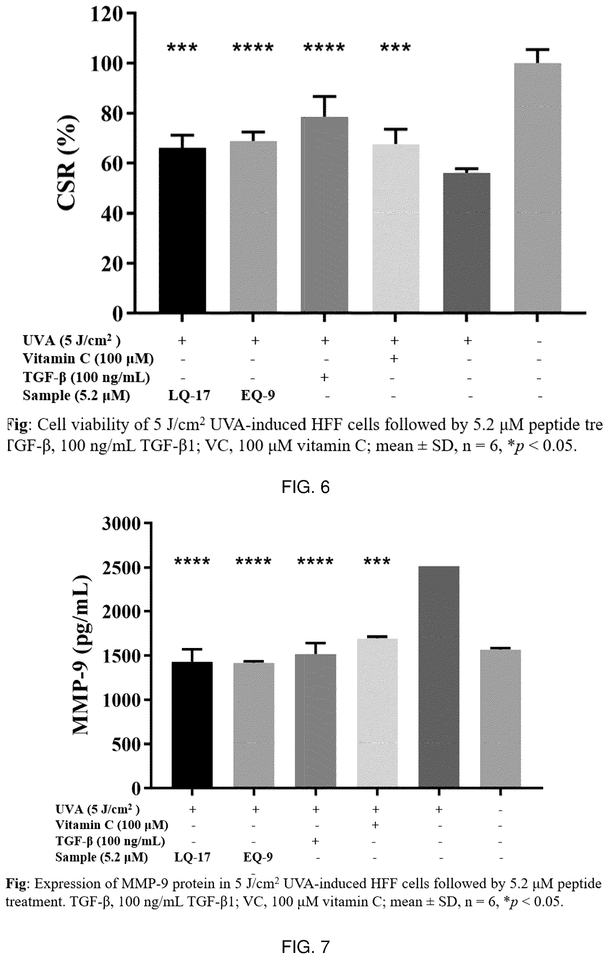

Experimental result shows that (see FIG 6): EQ-9 and LQ-17 may repair UVA-caused damage and increase cell viability.

### Example 5: Quantitative Detection Experiment for MMP-9 Expression

Human foreskin fibroblast HFF cells were subjected to resuscitation and passage. After a state of the cells was good upon microscopic examination, the cells were collected, and added to a 96-well plate according to a cell density of 2.0-2.5*10⁴/well. The cells were cultured in an incubator for 24h (37°C, 5% CO₂). Culture medium was discarded. Fresh culture medium was added. Induction was performed with a certain amount of UVA, wherein a total irradiation amount was controlled to be 5J/cm². Supernatant was discarded. A culture medium containing certain testing substances was added. After culturing for 24h, cell culture medium was collected, and centrifuged at 1000 g for 10 min at 4°C. A content of MMP-9 protein was measured using a corresponding kit.

Experimental result shows that (see FIG. 7): EQ-9 and LQ-17 may significantly reduce an MMP-9 expression amount of cells after ultraviolet damage (MMP-9 belongs to a matrix metalloprotease family and may degrade extracellular collagen).

By summarizing the above experimental results, it is indicated that EQ-9 bioactive peptide is a promising anti-aging ingredient with good safety, and has the effects of promoting cell growth, protecting cells from UVA damage and increasing production of collagen I that are equivalent to those of LQ-17. In terms of increasing the intracellular ATP content after UVA stimulation, EQ-9 is significantly superior to LQ-17. The EQ-9 bioactive peptide is shorter than LQ-17 in length, but has the same or better activity, and a lower production cost, thus having great commercial potential. In contrast, other truncated bodies, such as VQ-13 and LT-12, have poor overall effects.

### Example 6: EQ-9 Peptide Modification and Bioactivity Study

The inventors carried out detailed structural and functional researches on EQ-9, and the following bioactive peptides were obtained through modification.

**Table 3: Aggregation of Modified Derivative Peptides**

| No. | Name | Sequence |
|---|---|---|
| 1 | LQ-17 | LSDQVPDTESET**RILL**Q |
| 2 | EQ-9 | ESET**RILL**Q |
| 3 | EQ-9-1 | ESET**KILL**Q |
| 4 | DQ-9 | DSDA**KILI**Q |
| 5 | DQ-9-2 | DSDA**KLLI**Q |
| 6 | EE-9-1 | ETDT**RILV**E |
| 7 | EE-9-2 | ESEA**RIIL**E |
| 8 | DD-9 | DSES**KLVI**D |
| 9 | EQ-9-2 | ESET**EAVL**Q |
| 10 | EQ-9-3 | ESKI**RILL**Q |
| 11 | EL-6 | ET**RILL** |
| 12 | SL-6 | SET**RIL** |

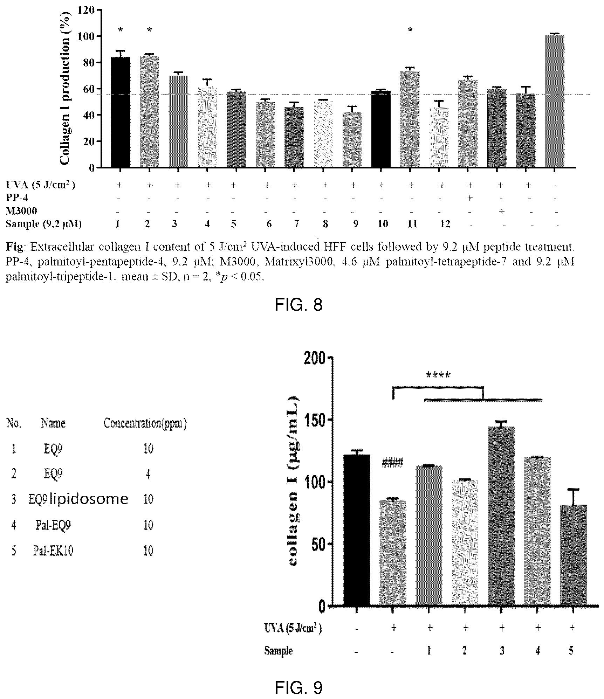

The above modified bioactive peptides were subjected to UVA damage repair experiment (experimental steps were the same as those in Example 3). Result is as shown in FIG. 8.

Result of LQ-17 truncation experiment shows that compared with VQ-13 and EQ-9, LT-12 almost completely loses activity, indicating that truncated RILLQ has an important impact on the activity. After further truncation of EQ-9, it is found that EL-6 retains better bioactivity, while SL-6 shows loss of activity, further demonstrating the importance of RILL region. After different point mutations on EQ-9, the bioactivity thereof is affected to some extent. By comparing EQ-9 and EQ-9-1, after R is mutated to K, the activity of the polypeptide is reduced. By comparing EQ-9 and EQ-9-2, after RIL is mutated to EAV, the activity of the polypeptide is completely lost, and even an inhibitory effect is displayed. It can thus be seen that the inventors have verified a core functional domain of the EQ-9 bioactive peptide through functional experiments, and upon comprehensive determination, EQ-9 has the best effect in combination with other LQ-17 truncated bodies with biological function.

### Example 7: EQ-9 Bioactive Peptide Palmitoylation Modification Effect

The EQ-9 bioactive peptide was modified as follows:

### 7.1 Palmitoylation Modification

Polypeptide was synthesized by a method of solid-phase synthesis, wherein concise steps were as follows: first, synthesizing Fmoc-AA1-Wang resin, treating the resin with a 20% piperidine solution dissolved in dimethylformamide (DMF) so as to unblock amino groups, and then treating with Fmoc-AA2 and 1-hydroxybenzotriazole (HOBt) to obtain Fmoc-AA2-AA1-Wang resin (note: AA1 and AA2 represent amino acids). The above process was repeated to generate final Fmoc-peptide-Wang resin. Finally, the amino groups were unblocked, and treated with palmitic acid and 1-hydroxybenzotriazole (HOBt) to obtain palm-peptide-Wang resin. Crude peptide was released from the resin using TFA methylene chloride water, and subsequently the crude peptide was purified by HPLC so to ensure purity thereof to be greater than 95%. Finally, the peptide was freeze-dried into polymorphic crystalline powder.

### 7.2 Liposome Encapsulated Delivery

Sodium surfactin, soybean lecithin, 1,3-propanediol, glycerol, 1,2-pentanediol, 1,2-hexanediol, etc. of different molar concentrations were dissolved with diethyl ether, and gradually dropwise added to an aqueous solution containing 1 mg/mL polypeptide (EQ-9), with a temperature of the aqueous solution being maintained at 50-60 °C. Mixture was continuously stirred for 2 h, to promote evaporation of diethyl ether. Microscopic examination was carried out to determine shape and size of liposomes. Sampling was carried out to determine encapsulation efficiency. Qualified liposomes were subjected to subsequent experiment.

EQ-9 peptide having undergone the above modification was subjected to the experiment of testing ability to repair UVA damage as described in Example 4 (experimental steps were substantially the same), wherein: 1) 10 ppm of EQ-9; 2) 4 ppm of EQ-9; 3) 10 ppm of EQ-9 liposomes; 4) 10 ppm of palmitoylated EQ-9; 5) EQ-9 derivative with C end being added with one lysine, called as EK-10-pal, on the basis of EQ-9-pal, with concentration of 10 ppm;

Experimental results (as shown in FIG. 9) are as follows:
1) 10 ppm of palmitoylated EQ9 may significantly improve the anti-aging efficacy, and even a dose of 4 ppm also may achieve certain effect;
2) 10 ppm of EQ9 also may significantly improve the anti-aging efficacy through liposome encapsulation; and
3) in contrast, EK10-pal has no anti-aging efficacy in this *in vitro* cell model.

The above description is merely for optional embodiments that are merely illustrative and do not limit combinations of features necessary to implement the present disclosure. Headings provided are not intended to limit various embodiments of the present disclosure. The terms such as "containing", "including" and "comprising" are not intended to be limiting. In addition, unless otherwise indicated, where there is no numeral modification, the plural form is included, and "or" and "alternatively" means "and/or". Unless otherwise defined herein, all technical and scientific terms used herein have the same meaning as commonly understood by those skilled in the art.

## Claims

1. A bioactive polypeptide, wherein an amino acid sequence of the bioactive polypeptide conforms to a following general formula:
R1-AA₁-AA₂-AA₃-AA₄-AA₅-AA₆-AA₇-AA₈-AA₉
wherein
AA₁ is selected from E and D or is a bond,
AA₂ is selected from S and T or is a bond,
AA₃ is selected from E, D, and K,
AA₄ is selected from T, A, S, and I,
AA₅ is selected from R, K, and E,
AA₆ is selected from I, L, and A,
AA₇ is selected from L, I, and V,
AA₈ is selected from L, I, and V,
AA₉ is selected from Q, E, and D or is a bond, and
R1 is selected from the group consisting of H, acetyl, tert-butanoyl, hexanoyl, 2-methylhexanone, cyclohexanecarboxyl, octanoyl, decanoyl, lauroyl, myristoyl, palmitoyl, stearoyl, oleoyl and linoleoyl.

2. The bioactive polypeptide according to claim 1, wherein AA₅ is R, AA₆ is I, AA₇ is L, and AA₈ is L.

3. The bioactive polypeptide according to claim 1, wherein the amino acid sequence thereof is selected from the group consisting of ESETRILLQ, ESETKILLQ, DSDAKILIQ, DSDAKLLIQ, ETDTRILVE, ESEARIILE, DSESKLVID, ESETEAVLQ, ESKIRILLQ, ETRILL, and SETRIL.

4. Use of the bioactive polypeptide according to any one of claims 1-3 in preparation of food, cosmetics, health care products or medicines having an anti-aging function.

5. Use of the bioactive polypeptide according to any one of claims 1-3 in preparation of food, cosmetics, health care products or medicines having an anti-ultraviolet damage function.

6. Use of the bioactive polypeptide according to any one of claims 1-3 in preparation of food, cosmetics, health care products or medicines having an anti-inflammatory function.

7. A derivative for synthesizing the bioactive polypeptide according to any one of claims 1-3, wherein the derivative is a polypeptide derivative obtained through modification on an amino acid side chain group, amino terminal or carboxyl terminal of the bioactive polypeptide according to any one of claims 1-3, comprising hydroxylation, carboxylation, carbonylation, methylation, acetylation, phosphorylation, esterification or glycosylation, meanwhile without affecting biological activity thereof.

8. A pharmaceutical composition, containing the bioactive polypeptide according to any one of claims 1-3, the derivative of the bioactive polypeptide according to claim 7, and at least one excipient or a cosmetically or pharmaceutically acceptable adjuvant.

9. The pharmaceutical composition according to claim 8, wherein the excipient comprises: hydroxyethyl cellulose, hydroxypropyl cellulose, hydroxypropyl methylcellulose, polyethylene glycol, polyacrylamide, polyacrylic acid, polyvinyl alcohol, dextran, ammonia, lactic acid, citric acid, pyrrolidone carboxylic acid, glycerol, urea, sorbitol, amino acids, lanolin, and petrolatum.

10. The pharmaceutical composition according to claim 8, wherein the adjuvant comprises: a solubilizing agent, a thickening agent, a gelling agent, a softening agent, an antioxidant, a suspending agent, a stabilizer, a foaming agent, a flavoring agent, a surfactant, water, an ionic or non-ionic emulsifier, a filler, a chelating agent, a chelating agent, a preservative, a vitamin, a blocking agent, a wetting agent, essential oil, a dye, and pigment.
